# EUROPEAN PATENT APPLICATION

(11) **EP 2 647 378 A1**
(43) Date of publication of application: **09.10.2013**
(21) Application number: 12163418.2
(22) Date of filing: 05.04.2012
(51) Int. Cl.: A61K 31/565, A61P 15/00

(54) **Steroid sulfatase inhibitor regimen for the treatment of endometriosis**

(71) Applicant: PregLem S.A., 1228 Plan-les-Ouates Geneva (CH)
(72) Inventor: Gotteland, Jean-Pierre, 1205 Genève (CH); Pohl, Olivier Martin, 74160 ARCHAMPS (FR); Loumaye, Ernest, 1223 COLOGNY (CH)
(74) Representative: reuteler & cie SA

(57) **Abstract**

The present invention is related to a dosage regimen of a steroid sulfatase inhibitor, E2MATE, for use in the prevention or treatment of endometriosis. The present invention further relates to a method for preventing or treating endometriosis.

## Description

### Field of the Invention

The present invention relates to a dosage regimen of a steroid sulfatase inhibitor, E2MATE or EMATE, for use in the prevention or treatment of endometriosis. The present invention further relates to a method for preventing or treating endometriosis.

### Background of the Invention

Endometriosis is characterized by the presence of endometrium-like tissue outside the uterus cavity (e.g. endometrial glands and stroma), most frequently in the peritoneal cavity. The incidence of endometriosis is difficult to quantify as women suffering from the disease are often asymptotic and imaging techniques present low sensitivities for diagnosis and thus endometriosis is a highly prevalent but highly underdiagnosed condition. There are an estimated 7 million endometriosis patients in the USA, 12-14 million endometriosis patients in Europe and estimated 80 million in the rest of world.

The primary method of diagnosis for endometriosis is visualization of endometriotic lesions by laparoscopy with or without biopsy confirmation. A commonly used staging of the disease is based on endometriotic lesion morphology which classifies the disease in four stages: stage I (minimal), stage II (mild), stage III (moderate) and stage IV (severe) (The Revised American Society for Reproductive Medicine. Classification of endometriosis. Fertil Steril., 1997; 67:817-821). Clinically, the severity of the disease would not necessarily follow the severity of the felt symptoms, i.e. a women with severe endometriosis (stage IV) may have few complaints such as pelvic pain, dysmenorrhea (cyclic pain menstruation), pain during intercourse, painful urination or defecation, dyspaneuria, subfertility, whereas those presenting a minimal disease stage (stage I) may have significant pain and subfertility or both.

The increased frequency or heaviness of menstrual flow is a clear risk factor for the development of this disorder and evidence of familial inheritance pattern of endometriosis has been described (Stefanson et al., 2002, Hum. Reprod., 17:555*;* Wheeler, 1992, Infertil. Reprod. Med. Clin. North Am., 3:545-9).

Among key components of the disease process, its estrogen-dependency character explains that it almost exclusively affects women of reproductive age (typically women between the ages of 15 and 50) since these have cycle-dependent high circulating estrogen levels. The biologically active estrogen, estradiol, aggravates the pathological process (e.g. inflammation and growth) and the symptoms (e.g. pain) associated with endometriosis. Main biologically significant sources of estrogens in endometriosis are the ovary and the endometriotic lesion itself. Both, circulating levels of estrogens from the ovary as well as local productions of estrogens in the endometriotic lesions themselves are important aspects of this disease, but the significance of each estrogen source may vary from patient to patient. Endometric lesions are progesterone-resistant, nevertheless progestins can be used to counteract the estrogenic effects of estradiol on the endometrium causing initial decidualization and subsequent endometrial atrophy.

Treatment for endometriosis depends on women's specific symptoms, severity of symptoms and location of the endometriotic lesions but it generally primarily aims at minimizing disease and associated symptoms. For women suffering from mild pain, the usual treatment is non-steroidal anti-inflammatory drugs (NSAIDs) such as selective cyclo-oxygenase-2 inhibitors (COX-2 inhibitors), combined oral contraceptives (COCs) or progestins where combined oral contraceptives (COCs) or progestins are considered as the first-line option as an alternative to surgery and as post-operative adjuvant measure. For women suffering from moderate to severe pain, laparoscopy is the most common diagnosis and treatment method.

COX-2 inhibitors have been reported to reduce dysmenorrhea and pelvic pain but due to the cardiovascular risk associated with their long-term use this class of substances should be used at the lowest doses and for the shortest duration possible (Jones, 2005, Ann. Pharmacother, 39, 1249).

When analgesics like cyclo-oxygenase-2 inhibitors are not efficacious or their prescription in certain patients is not possible due to their side effects, treatments for endometriosis aim at reducing or suppressing menstruation and oestrogen production by the ovary. This is achieved by androgens like danazol, progestins, combined oral contraceptive pills or GnRH agonists. Combined oral contraceptives act by inhibiting gonadotropin release, decrease menstrual flow and decidualizing implants and lead to a suppression of ovulation and relief of endometriosis-related pain (Vercellini et al. 1993, Fertil Steril., 60(1):75-9). Progesterone receptor ligand agonists (progestin) are widely used in the treatment of various gynecological disorders, including endometriosis by antagonizing estrogenic effects on the endometrium. GnRH agonists block ovarian steroid secretion and result in serum levels of estradiol lower than 30 pg/ml (Barbieri et al. 1992, Am. J. Obstet. Gynecol., 166; 740-5).

There are, however, many side effects related to those treatments, e.g. the use of GnRH agonists is limited to 6 months because of observed adverse effects on bone mineral density and treatment with danazol is also limited because of its androgenic side-effects. A GnRH agonist may be used for longer periods in combination with the daily administration of a progestin, norethindrone acetate (NETA) for protecting against bone loss induced by GnRH agonist treatment (survey, 2002, Obstet. Gynecol., 99(5 Pt 1): 709-19). However, in patients responding to treatment with GnRH agonists, symptom recurrence is reported in a majority of the patients within 5 years of treatment cessation. Chronic use of progestins is associated sometimes with unacceptable side effects such as breakthrough bleeding, spotting change in menstrual flow, amenorrhea, edema, changes in weight (decreases, increases), changes in the cervical squamo-columnar junction and cervical secretions, cholestatic jaundice, rash (allergic) with and without pruritus, melasma or chloasma, clinical depression, acne, breast enlargement/tenderness, headache/migraine, urticarial, abnormalities of liver tests (i.e., alanine transaminase (ALT), aspartate aminotransferase (AST), Bilirubin), decreased HDL cholesterol and increased LDL/HDL ratio, mood swings, nausea, insomnia, anaphylactic/anaphylactoid reactions, thrombotic and thromboembolic events (e.g., deep vein thrombosis, pulmonary embolism, retinal vascular thrombosis, cerebral thrombosis and embolism),optic neuritis (which may lead to partial or complete loss of vision). In addition, COC treatment may lead to an increased risk of venous thromboembolic disease, myocardial infarction, ischemic stroke or benign liver tumors *(*Wiegratz et al., 2011, Dtsch. Arztebl. Int., 108(28-29): 495-506).

Currently available treatments of endometriosis based on non-steroidal anti-inflammatory drugs (NSAIDS) or hormonal treatments like danazol, progestins or GnRH agonists alleviate endometriosis symptoms in only less than half of the patients.

Steroid sulfatase or steryl sulfatase (STS) is a microsomal enzyme catalyzing the hydrolysis of aryl and alkyl steroid sulfates *(*Reed et al., 2005, Endocr. Rev., 26(2), 171-202*)* which has an essential role in regulating the formation of biologically active steroids. It is notably crucial for the local production of active estrogens and androgens through the conversion of their systemic circulating sulphated precursors, namely estrone sulphate (E1S) and dehydroepiandrosterone sulphate (DHEAS), respectively. Both estrone and dehydroepiandrosterone can be converted to steroids with estrogenic properties (i.e estradiol and androstenediol). STS mRNA expression was reported to be five-fold higher in ovarian endometriosis compared to normal endometrium (Smuc et al., 2007, Gynecol. Endocrinol., 23:105-111). Moreover, it was reported that STS activity has been detected in both eutopic and ectopic endometrium and that STS activity in endometriotic implants has been shown to correlate with the severity of the disease *(*Purohit et al., 2008, Hum. Reprod., 23(2), 290-7*).* STS inhibitor COUMATE (or STX64 or 667) has shown to be effective in blocking STS activity in both eutopic and ectopic tissues *(Purohit et al*., *2008, supra*).

Estradiol-3-o-sulfamate (E2MATE) is readily transported and absorbed in the gut into its oxidative metabolite Estrone-3-o-sulfamate (EMATE) and both compounds have shown to be active STS inhibitors (Numazawa et al., 2006, Steroids, 71(5):371-9).

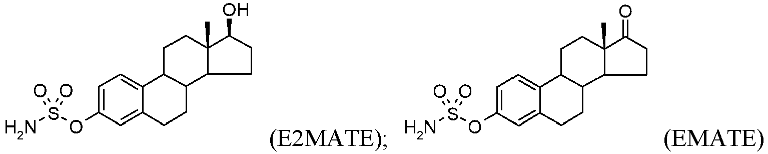

E2MATE was used at a daily oral dose of 0.5 or 1 mg/kg for 21 days in mice in a model of endometriosis where endometriosis-like lesion formation is induced. STS activity inhibition in the uterus or in induced lesions reached a value not higher than 36% at the highest dose and lesion weight and size were decreased at the end of the treatment without a reduction of the number of lesions (Colette et al., 2010, Hum. Reprod., 0(0), 1-9). Further, some stromal oedema was observed in the histology of the uterus which is indicative of some estrogenic activity of E2MATE.

It has been described that administration of a steroid sulfatase inhibitor to adult female non-human primates and pre-menopausal women disturb ovulation and the menstrual cycle resulting in delayed or absence of ovulation and delayed or absence of menstruation (WO 2009/037539) which are undesired side effects that would reduce quality of life of patients. Further, those effects have been reported to be associated with fluctuating and persistent estrogen levels which can partially counteract the therapeutic benefit of the local inhibition of STS activity by exposing the estrogen-dependant tissue to high levels of circulating estrogens. In order to palliate this reported undesired effect, WO 2009/037539 discloses the co-administration in pre-menoposal women with functional ovaries of a STS inhibitor and a therapeutically effective amount of a compound selected from the group comprising a progesterone agonist (progestin), an oral combined estrogen and progestin contraceptive and/or a GnRH analog.

Therefore, currently available treatments of endometriosis are not fully optimal due to their side effects and of the non-responding population of patients. Thus, there remain significant unmet needs for efficient, safe and better long-term therapies for treating endometriosis and its symptoms.

### Summary of the Invention

The present invention is based on the unexpected finding of the benefits of a STS inhibitor regimen, E2MATE or EMATE at a loading dose, allowing the nearly complete and long-lasting inhibition of STS in blood cells and in endometrium tissue within a short period of treatment. The invention is further based on the additional unexpected finding that the regimen of the invention allows the use of a lower dose of the STS inhibitor than the loading dose (maintenance dose) after a first period of treatment if longer treatment is needed, while maintaining the nearly complete and long-lasting inhibition of STS. Further surprisingly, it was found that the regimen according to the invention results in unmodified circulating estradiol levels and does not induce any perturbation of the ovarian cycle parameters. Finally, it was found that such a regimen may be advantageously combined with the co-administration of a progestin which further decreases STS activity in the endometrium tissue in a synergetic manner and therefore further potentiates the activity of the STS inhibitor. Notably, the invention is related to new dose regimen of the steroid sulfatase inhibitor estradiol sulfamate E2MATE or EMATE or a pharmaceutical formulation thereof, optionally in combination with a progestin useful in the inhibition and/or reduction of endometriosis symptoms and to prevent the occurrence of said symptoms (e.g. to avoid recurrence after surgery).

An embodiment described herein provides an improved dosing regimen for E2MATE or EMATE in the treatment and prevention of endometriosis as defined in the claims.

An additional embodiment of the invention provides a use of E2MATE or EMATE for the preparation of a pharmaceutical formulation for the treatment and prevention of endometriosis wherein perturbation of the ovarian cycle parameters are reduced or avoided and allowing further use of E2MATE or EMATE as defined in the claims.

In a first aspect, the invention provides E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks and wherein the total dose of E2MATE or EMATE reached at the end of the loading period is from about 8 mg to about 30 mg.

In a second aspect, the invention provides a method of treatment or prophylaxis of endometriosis comprising the administration in a patient in need thereof of E2MATE or EMATE, any pharmaceutically acceptable salts or complexes thereof or a pharmaceutical formulation thereof, for at least one loading period lasting for about four weeks and wherein the total dose of E2MATE or EMATE reached at the end of the loading period is from about 8 mg to about 30 mg.

In a third aspect, the invention provides a method of treatment of endometriotic lesions and/or endometriotic pain comprising the administration in a patient in need thereof of E2MATE or EMATE any pharmaceutically acceptable salts or complexes thereof or a pharmaceutical formulation thereof, for at least one loading period lasting for about four weeks and wherein the total dose of E2MATE or EMATE reached at the end of the loading period is from about 8 mg to about 30 mg.

In a fourth aspect, the invention provides a pharmaceutical pack or kit comprising one or more containers filled with E2MATE or EMATE, any pharmaceutically acceptable salts or complexes thereof or a pharmaceutical formulation thereof, wherein each container contains a unit dose necessary for a treatment lasting about four weeks and wherein the total dose of E2MATE or EMATE of said treatment is from about 8 mg to about 30 mg.

In a fifth aspect, the invention comprises a pharmaceutical formulation comprising E2MATE or EMATE, any pharmaceutically acceptable salts or complexes thereof and norethindrone (norethisterone) acetate (NETA), any pharmaceutically acceptable salts or complexes thereof and a pharmaceutically acceptable carrier, diluent or excipient thereof. Other features and advantages of the invention will be apparent from the following detailed description.

### Description of the figures

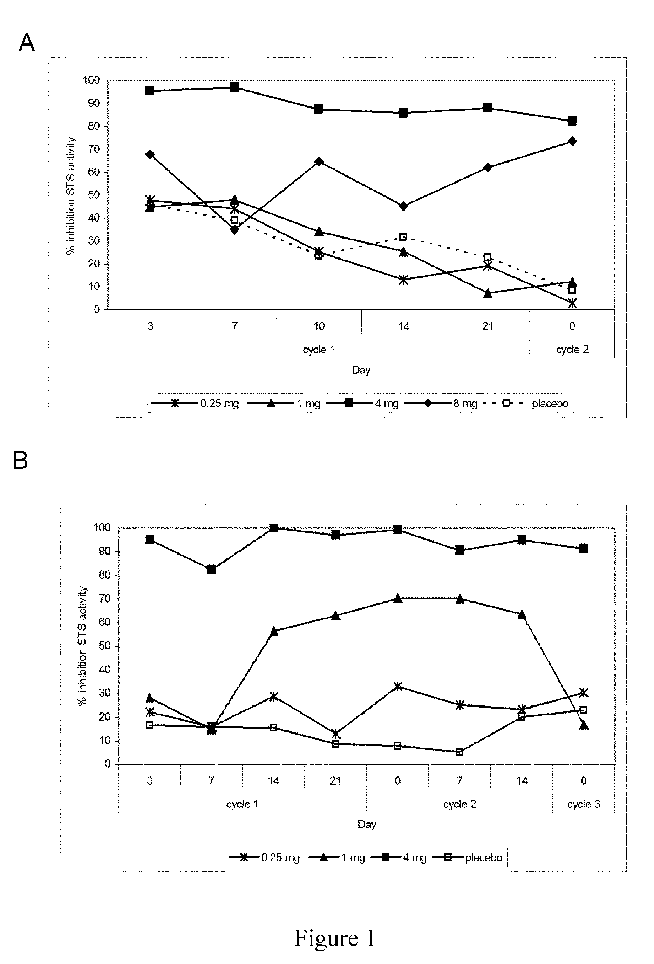

**Figure 1** shows the percentage Steroid Sulfatase Inhibition in PBMCs after administration of E2MATE as single dose **(A);** multiple dose **(B)** as described in Example 1.

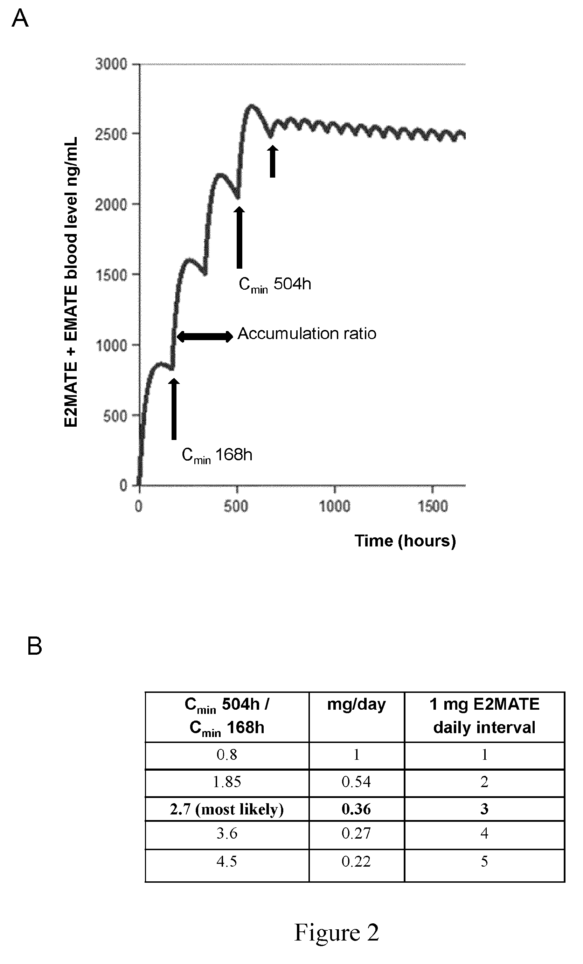

**Figure 2** represents the pharmacokinetic behavior of E2MATE based on the pharmacokinetic data from the study of the Example 1 where is calculated the accumulation ratio of E2MATE + EMATE **(A)** and the corresponding daily dose (mg/day) and the daily interval for a dose of 1 mg of E2MATE which are necessary for maintaining the E2MATE + EMATE blood levels obtained at the end of the loading period **(B),** as described in Example 3.

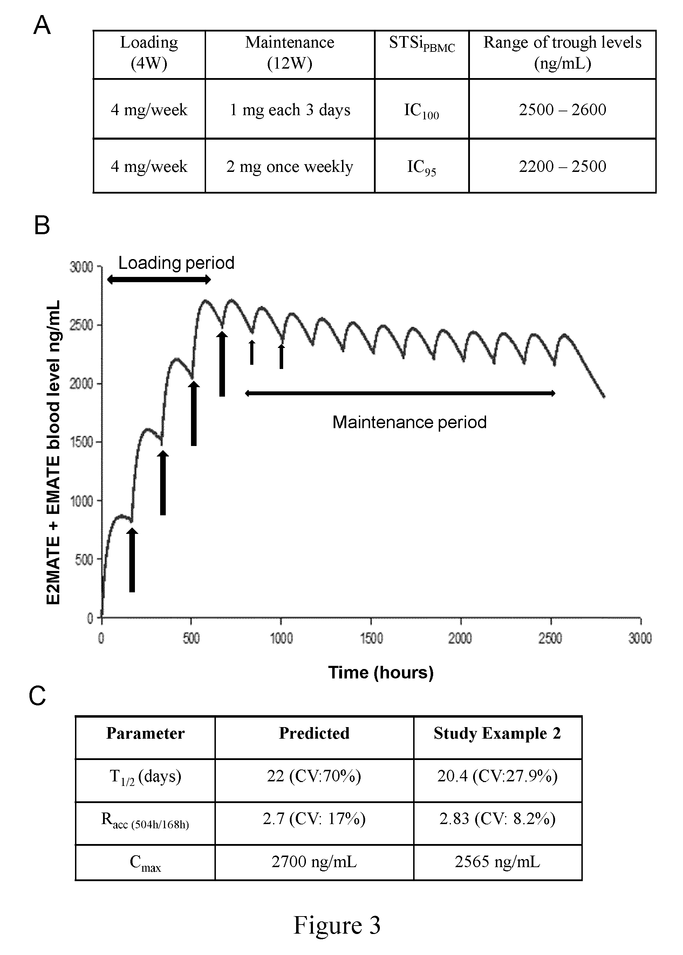

**Figure 3** shows pharmacokinetic parameters derived from the model of Example 3 for E2MATE. **A:** blood levels of E2MATE + EMATE (ng(mL) necessary to maintain a STS activity in PBMCs of 100% (IC₁₀₀) or 95% (IC₉₅) at the end of the loading period and the corresponding regimen (dose and daily interval) of E2MATE necessary for maintaining such levels; **B**: modelised pharmacokinetic behavior of E2MATE for a treatment regimen according to the invention with a loading treatment period of 4 mg/week during 4 weeks with E2MATE and a maintenance treatment period of 2 mg/week during 12 weeks as described in Example 3; **C:** comparison of the predicted values for pharmacokinetic parameters based on the pharmacokinetic model as described in Example 3 with the measured values in the study of Example 2.

### Detailed Description of the invention

The following paragraphs provide definitions of the various chemical moieties that make up the compounds according to the invention and are intended to apply uniformly throughout the specification and claims, unless an otherwise expressly set out definition provides a broader definition.

In the present context, the term "progestin" (also sometimes referred to as "gestagen" or "progestogen") covers synthetic compounds which are progesterone receptor agonists. The term is further meant to encompass all isomeric and physical forms of the progestins including hydrates, solvates, salts and complexes, such as complexes with cyclodextrins. Specific examples of progestins include, but are not limited to, progestins such as levo-norgestrel, norgestrel, norethindrone (norethisterone), dienogest, norethindrone (norethisterone) acetate (NETA), ethynodiol diacetate, dydrogesterone, medroxyprogesterone acetate, norethynodrel, allylestrenol, lynestrenol, quingestanol acetate, medrogestone, norgestrienone, dimethisterone, ethisterone, chlormadinone acetate, megestrol, promegestone, desogestrel, 3-keto-desogestrel, norgestimate, gestodene, tibolone, cyproterone acetate, dienogest, drospirenone, nestorone, nomegestrol acetate, trimegestone and nonsteroidal progesterone receptor (PR) agonist such as tanaproget. According to a specific embodiment, a progestin according to the invention is selected from dienogest, norethindrone (norethisterone) and norethindrone (norethisterone) acetate (NETA).

The term "total dose" or "cumulative dose" refers to the total dose of E2MATE or EMATE administered during the treatment period, i.e. the dose reached at the end of the treatment period calculated by adding the individual doses (e.g. daily, weekly etc..). For example, the total dose reached at the end of the loading period of a regimen according to the invention may be reached by a dose of about 2 mg to about 7.5 mg per week or of about 0.8 mg to about 3 mg every three days or a daily dose of about 0.3 mg to about 1 mg or even a single dose of 8 mg to about 30 mg. For example, a total dose reached at the end of the loading period of a regimen according to the invention encompasses a dose of about 5 mg per week or about 2 mg every three days or a daily dose of about 0.7 mg or even a single dose of 20 mg. As another example, the total dose reached at the end of the loading period of a regimen according to the invention may be reached by a dose of about 3 mg per week or about 1.3 mg every three days or a daily dose of about 0.4 mg or even a single dose of about 12 mg. In a particular embodiment, the total dose reached at the end of the loading period of a regimen according to the invention may be reached by a dose of about 4 mg per week or about 2 mg every three days or a daily dose of about 0.6 mg or even a single dose of about16 mg.

The term "pharmaceutically acceptable salts or complexes" refers to salts or complexes of the steroid sulfatase inhibitors or progestin of the invention. Examples of such salts include, but are not restricted, to base addition salts formed by reaction with organic or inorganic bases such as hydroxide, carbonate or bicarbonate of a metal cation such as those selected in the group consisting of alkali metals (sodium, potassium or lithium), alkaline earth metals (e.g. calcium or magnesium), or with an organic primary, secondary or tertiary alkyl amine. Amine salts derived from methylamine, dimethylamine, trimethylamine, ethylamine, diethylamine, triethylamine, morpholine, N-Me-D-glucamine, N,N'-bis(phenylmethyl)-1,2-ethanediamine, tromethamine, ethanolamine, diethanolamine, ethylenediamine, N-methylmorpholine, procaine, piperidine, piperazine and the like are contemplated being within the scope of the instant invention.

Also comprised are salts which are formed from to acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, and poly-galacturonic acid.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions. In particular, efficacy of a treatment according to the invention can be measured based on changes in the course of disease in response to a use or a method according to the invention. For example, the efficacy of a treatment of endometriosis or endometriotic pain can be measured by monitoring the number or size of endometriotic lesions detected by imaging by sonography, Magnetic Resonance Imaging, Diagnostic Laparoscopy, and by the serial measurement of appropriate markers such as but not limited to e.g. CA125 or CA19. Effective treatment is indicated by reduction in lesion number or size, and diminishing levels or maintenance of basal levels of at least one specific marker. Successful outcome results are for example a decrease of pain, and/or a decreased risk of symptom recurrence after treatment including surgery.

A "treatment" according to the invention comprises at least a loading period according to the invention. Optionally, a treatment according to the invention further comprises a maintenance treatment at the end of the loading period. Optionally, a progestin may be administered during the loading and/or maintenance treatment.

The term "loading treatment" or "loading period" consists in at least one loading period where E2MATE or EMATE is administered. A loading period lasts up to about four weeks. A "maintenance treatment" or "maintenance period" consists in least one maintenance period wherein E2MATE or EMATE is administered at a dose of about 1 to 3 mg/week. Typically, a maintenance period according to the invention lasts for at least about 4 weeks to about several months such as for example for about 4 weeks to about 36 months, about 24 months, such as from about two to about six months, for example for about 12 weeks. The term "recurrence" involves endometriotic symptoms such as recurrence of pelvic pain or recurrence of endometriotic lesions.

In particular, the methods, uses, formulations and compositions according to the invention are useful in the treatment of endometriosis and/or in the prevention of evolution of endometriotic condition into an advanced or severe stage in patients with early stage endometriosis, thereby improving the staging of endometriosis.

The term "subject" as used herein refers to a mammalian subject, and most preferably a human patient, who is suffering from endometriosis or at risk of developing endometriosis at risk of suffering from endometriosis recurrence. Typically, subjects at risk of developing endometriosis include women of reproductive age with first degree relatives presenting endometriosis or who have been exposed *in utero* to stilbenoids or similar endocrine disrupting compounds. Other risk factors comprise an obstructive malformation of the uterus, early onset of menarche, short menstrual cycles or abundant or painful menses.

### Regimen

In a first embodiment, the invention provides E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks and wherein the total dose of E2MATE or EMATE reached at the end of the loading period is from about 8 mg to about 30 mg.

In a further embodiment, the invention provides a dosage regimen according to the invention wherein the total dose of E2MATE or EMATE reached at the end of the loading period is from about 8 mg to about 20 mg.

In another further embodiment, the invention provides a dosage regimen according to the invention wherein the total dose of E2MATE or EMATE reached at the end of the loading period is from about 8 mg to about 16 mg.

In another further embodiment, the invention provides a dosage regimen according to the invention wherein the total dose of E2MATE or EMATE reached at the end of the loading period is about 16 mg.

In a further embodiment, the invention provides a dosage regimen according to the invention wherein E2MATE or any pharmaceutically acceptable salts or complexes thereof or a pharmaceutical formulation thereof is to be administered.

In a further embodiment, the invention provides a dosage regimen according to the invention wherein EMATE or any pharmaceutically acceptable salts or complexes thereof or a pharmaceutical formulation thereof is to be administered.

In a further embodiment, the invention provides a dosage regimen according to the invention wherein E2MATE or EMATE, any pharmaceutically acceptable salts or complexes thereof or a pharmaceutical formulation thereof is to be administered orally.

In a further embodiment, the invention provides a dosage regimen according to the invention wherein E2MATE or EMATE, any pharmaceutically acceptable salts or complexes thereof or a pharmaceutical formulation thereof is to be administered daily during the loading period.

In a further embodiment, the invention provides a dosage regimen according to the invention wherein E2MATE or EMATE, any pharmaceutically acceptable salts or complexes thereof or a pharmaceutical formulation thereof is to be administered every two or three days during the loading period.

In another further embodiment, the invention provides a dosage regimen according to the invention wherein E2MATE or EMATE, any pharmaceutically acceptable salts or complexes thereof or a pharmaceutical formulation thereof is to be administered once a week during the loading period.

In another further embodiment, the invention provides E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks at a dose of about 4 mg/week.

In a further embodiment, the invention provides a dosage regimen according to the invention wherein E2MATE or EMATE, any pharmaceutically acceptable salts or complexes thereof or a pharmaceutical formulation thereof is to be administered once during the loading period at a single dose of 16 mg.

In another further embodiment, the invention provides E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks and wherein the total dose of E2MATE or EMATE reached at the end of the loading period is from about 8 mg to about 30 mg (such as from about 8 mg to 20mg, typically from about 8 to about 16 mg) and is to be administered after the end of the loading period for at least one maintenance period at a dose of E2MATE or EMATE of 1 to about 3 mg/week.

In a further embodiment, the invention provides a dosage regimen according to the invention wherein E2MATE or EMATE, any pharmaceutically acceptable salts or complexes thereof or a pharmaceutical formulation thereof is to be administered daily during the maintenance period.

In a further embodiment, the invention provides a dosage regimen according to the invention wherein E2MATE or EMATE, any pharmaceutically acceptable salts or complexes thereof or a pharmaceutical formulation thereof is to be administered every two or three days during the maintenance period.

In another further embodiment, the invention provides a dosage regimen according to the invention wherein E2MATE or EMATE, any pharmaceutically acceptable salts or complexes thereof or a pharmaceutical formulation thereof is to be administered once a week during the maintenance period.

In another further embodiment, the invention provides a dosage regimen according to the invention wherein the maintenance period lasts for about 4 weeks to about thirty-six months.

In another further embodiment, the invention provides a dosage regimen according to the invention wherein the maintenance period lasts for about 4 weeks to about twenty-four months.

In another further embodiment, the invention provides a dosage regimen according to the invention wherein the maintenance period lasts for about 4 weeks to about twelve months.

In another further embodiment, the invention provides a dosage regimen according to the invention wherein the maintenance period lasts for about 4 weeks to about six months.

In another further embodiment, the invention provides a dosage regimen according to the invention wherein the maintenance period lasts for about 4 to 12 weeks.

In another further embodiment, the invention provides E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks at a dose of about 4 mg/week and is to be administered at the end of the loading period for at least one maintenance period lasting for about 4 to 12 weeks at a dose of about 1 to 3 mg/week (e.g. 2 mg/week).

In a further embodiment, E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for a maintenance period starting less than 24 days, typically less than 21 days after the end of the loading period.

In another further embodiment, E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for a maintenance period immediately after the end loading period (i.e. without any treatment discontinuation).

In another further embodiment, a progestin, any pharmaceutically acceptable salts or complexes thereof or a pharmaceutical formulation thereof is to be administered in combination with E2MATE or EMATE, any pharmaceutically acceptable salts or complexes thereof or a pharmaceutical formulation thereof in a regimen according to the invention.

In another further embodiment, a progestin is to be administered during the loading and/or maintenance period in combination with E2MATE or EMATE, any pharmaceutically acceptable salts or complexes thereof or a pharmaceutical formulation thereof in a regimen according to the invention wherein the daily dose of progestin during the treatment is of about 0.05 mg to about 100 mg.

In another further embodiment, a progestin is to be administered during the loading and/or maintenance period at a daily dose of about 0.05 to about 100 mg (such as from about 0.1 to about 50 mg, in particular from about 1 mg to about 20 mg, more particularly of about 1 mg to about 10 mg (e.g. about 5 mg or about 10 mg)) in combination with E2MATE or EMATE, any pharmaceutically acceptable salts or complexes thereof or a pharmaceutical formulation thereof in a regimen according to the invention.

In another further embodiment, norethindrone (norethisterone) acetate (NETA) any pharmaceutically acceptable salts or complexes thereof or a pharmaceutical formulation thereof is to be administered in combination with E2MATE or EMATE, any pharmaceutically acceptable salts or complexes thereof or a pharmaceutical formulation thereof in a regimen according to the invention.

In another further embodiment, norethindrone (norethisterone) acetate (NETA) any pharmaceutically acceptable salts or complexes thereof or a pharmaceutical formulation thereof is to be administered in combination with E2MATE or EMATE, any pharmaceutically acceptable salts or complexes thereof or a pharmaceutical formulation thereof in a regimen according to the invention at a daily dose of about 5 mg/day.

In another further embodiment, norethindrone (norethisterone) acetate (NETA) any pharmaceutically acceptable salts or complexes thereof or a pharmaceutical formulation thereof is to be administered in combination with E2MATE or EMATE, any pharmaceutically acceptable salts or complexes thereof or a pharmaceutical formulation thereof in a regimen according to the invention at a daily dose of about 10 mg/day.

In another further embodiment, the invention provides E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks at a dose of about 4 mg/week and is to be administered at the end of the loading period at a dose of about 2 mg/week during about four to about twelve weeks and wherein norethindrone (norethisterone) acetate is administered at a daily dose of about 5 to about 10 mg/day during the loading and maintenance periods.

### Compositions

E2MATE or EMATE or progestins according to the invention may be administered in the form of pharmaceutical compositions useful for the prophylaxis or treatment of endometriosis.

E2MATE or EMATE or progestins any pharmaceutically acceptable salts or complexes thereof or a pharmaceutical formulation thereof can be prepared from readily available starting materials using methods and procedures known from the skilled person. It will be appreciated that where typical or preferred experimental conditions (i.e. reaction temperatures, time, moles of reagents, solvents etc.) are given, other experimental conditions can also be used unless otherwise stated.

Pharmaceutical compositions of the invention may further comprise one or more pharmaceutically acceptable additional ingredient(s), such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily/weekly dosage range to be employed. Compositions according to the invention are preferably oral formulations.

Compositions of this invention may also be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 *of* Remington's Pharmaceutical Sciences, 21st Edition, 2005, University of the Sciences in Philadelphia, Lippincott Williams & Wilkins*,* which is incorporated herein by reference.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum,* and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the *stratum corneum*. The compounds of this invention can also be administered in sustained release forms or from sustained release drug delivery systems. A description of representative sustained release materials can also be found in the incorporated materials in *Remington's Pharmaceutical Sciences.*

In another further embodiment, in a pharmaceutical pack or kit according to the invention a notice for use is associated with such container(s).

In another further embodiment, the invention also relates to a pack or kit according to the invention comprising E2MATE or EMATE and separately therein a progestin.

In another further embodiment, the invention also relates to a pack or kit according to the invention comprising E2MATE or EMATE and separately therein NETA.

The pharmaceutical pack or kit according to the invention is useful in a regimen or treatment according to the invention.

### Mode of administration

Compositions of this invention may be administered in any manner, including, but not limited to, orally, parenterally, sublingually, transdermally, vaginally, intrauterine, rectally, transmucosally (e.g. sublingually or intra-vaginally or intrauterine), topically, via inhalation, via buccal or intranasal administration, or combinations thereof Parenteral administration includes, but is not limited to, intravenous, intra-arterial, intra-peritoneal, subcutaneous (e.g. depot injection), intramuscular, and intra-articular. The compositions of this invention may also be administered in the form of an implant, which allows slow release of the compositions as well as a slow controlled i.v. infusion. In a preferred embodiment, E2MATE or EMATE or progestin, any pharmaceutically acceptable salts or complexes thereof or a pharmaceutical formulation thereof according to the invention are administered orally.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, E2MATE or EMATE, any pharmaceutically acceptable salts or complexes thereof or a pharmaceutical formulation thereof can be administered alone or co-administered in combination with a co-agent useful in the prophylaxis or treatment of endometriosis, e.g. for example a co-agent selected from GnRH antagonists, Selective Estrogen Receptor modulators (SERMs) such as those described in Cano et al., 2000, Human Reprod. Update, 6(3), 244-254*,* E2 receptor beta agonists, Suppressing Steroids (e.g. Danocrine ®), aromatase inhibitors and progestins.

According to a particular embodiment of the invention, E2MATE or EMATE, any pharmaceutically acceptable salts or complexes thereof or a pharmaceutical formulation thereof is administered in combination with a progestin.

According to a further embodiment of the invention, E2MATE or EMATE, any pharmaceutically acceptable salts or complexes thereof or a pharmaceutical formulation thereof is administered in combination with norethisterone (NET) or norethindrone (norethisterone) acetate.

The invention encompasses the administration of E2MATE or EMATE, any pharmaceutically acceptable salts or complexes thereof or a pharmaceutical formulation thereof to an individual prior to, simultaneously or sequentially with other therapeutic regimens or co-agents useful in the treatment of endometriosis (e.g. multiple drug regimens), in a therapeutically effective amount. E2MATE or EMATE, any pharmaceutically acceptable salts or complexes thereof or a pharmaceutical formulation thereof that are administered simultaneously with said co-agents can be administered in the same or different composition(s) and by the same or different route(s) of administration.

According to another embodiment of the invention, is provided a combined pharmaceutical formulation comprising E2MATE or EMATE, any pharmaceutically acceptable salts or complexes thereof and norethindrone (norethisterone) acetate (NETA), any pharmaceutically acceptable salts or complexes thereof and a pharmaceutically acceptable carrier, diluent or excipient thereof

According to a further embodiment of the invention, is provided a combined pharmaceutical formulation comprising E2MATE or EMATE at a dosage of about 0.3 to about 1 mg, any pharmaceutically acceptable salts or complexes thereof and norethindrone (norethisterone) acetate (NETA) at a dosage of 1 to about 20 mg, any pharmaceutically acceptable salts or complexes thereof and a pharmaceutically acceptable carrier, diluent or excipient thereof.

According to another further embodiment of the invention, is provided a combined pharmaceutical formulation comprising E2MATE or EMATE at a dosage of about 0.3 to about 0.6 mg, any pharmaceutically acceptable salts or complexes thereof and norethindrone (norethisterone) acetate (NETA) at a dosage of 1 to about 10 mg, any pharmaceutically acceptable salts or complexes thereof and a pharmaceutically acceptable carrier, diluent or excipient thereof. Typically, such a combined formulation may be useful in the loading period of a regimen according to the invention.

According to a further embodiment is provided a use of a combined pharmaceutical formulation according to the invention for the prophylaxis or treatment of endometriosis.

### Patients

Patients according to the invention are patients suffering from endometriosis.

In a further embodiment, patients according to the invention are patients suffering from mild endometriotic pain.

In another further embodiment, patients according to the invention are patients suffering from moderate to severe endometriotic pain.

In another further embodiment, patients according to the invention are patients which have been subjected to a treatment of endometriosis by surgery.

### Use according to the invention

In one embodiment, the invention provides a E2MATE or EMATE any pharmaceutically acceptable salts or complexes thereof or a pharmaceutical formulation thereof, for use in a method of treatment or prophylaxis of endometriosis or a method of treatment of endometriotic lesions and/or endometriotic pain according to the invention wherein said E2MATE or EMATE, any pharmaceutically acceptable salts thereof or a pharmaceutical formulation thereof are to be administered according to a regimen according to the invention.

In another embodiment, the invention provides a method of treatment or prophylaxis of endometriosis comprising the administration in a patient in need thereof of E2MATE or EMATE any pharmaceutically acceptable salts or complexes thereof or a pharmaceutical formulation thereof, for at least one loading period lasting for about four weeks and wherein the total dose of E2MATE or EMATE reached at the end of the loading period is from about 8 mg to about 30 mg.

In another embodiment, the invention provides a method of treatment of endometriotic lesions and/or endometriotic pain comprising the administration in a patient in need thereof of E2MATE or EMATE any pharmaceutically acceptable salts or complexes thereof or a pharmaceutical formulation thereof, for at least one loading period lasting for about four weeks and wherein the total dose of E2MATE or EMATE reached at the end of the loading period is from about 8 mg to about 30 mg.

In another embodiment, a method of treatment or prophylaxis of endometriosis or a method of treatment of endometriotic lesions and/or endometriotic pain according to the invention further comprises, after the end of the loading period, the administration in a patient in need thereof of E2MATE or EMATE any pharmaceutically acceptable salts or complexes thereof or a pharmaceutical formulation thereof for at least one maintenance period lasting for about four to about twelve weeks at a dose of about 1 to 3 mg/week (e.g. 2 mg/week).

In another further embodiment, the invention provides a method according to the invention wherein the total dose of E2MATE or EMATE reached at the end of the loading period is from about 8 mg to about 20 mg.

In another further embodiment, the invention provides a method according to the invention wherein the total dose of E2MATE or EMATE reached at the end of the loading period is from about 8 mg to about 16 mg.

In another further embodiment, the invention provides a method according to the invention wherein the total dose of E2MATE or EMATE reached at the end of the loading period is about 16 mg.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

### The following abbreviations refer respectively to the definitions below:

**RIA** (radio immunoassay), **ALT** (alanine transaminase), **AST** (aspartate aminotransferase), **D4A** (androstenedione), **DHEAS** (dehydroepiandrosterone sulphate), **E1S** (Oestrone sulphate), **E2** (serum estradiol), **EOS** (end of study), **FSH** (Follicle-stimulating hormone), **P4** (progesterone), **HDL** (High density lipoprotein), **LC-MS/MS** (Liquid chromatography-masss spectrometry), **LDL** (Low density lipoprotein), **LH** (Luteinizing hormone), **NET** (norethisterone), **PBMC** (Peripheral Blood Mononuclear Cell), **STS** (Steroid sulfatase), **STS-I** (Steroid sulfatase inhibitor), **T** (testosterone), **TVUS** (transvaginal ultrasound).

### Example 1: Dosage regimen with one loading period

A dosage regimen of E2MATE according to the invention was carried out and compared to other dosages in healthy pre-menopausal women in order to support the benefit of a regimen according to the invention, notably in term of STS activity inhibition, circulating estradiol levels and ovarian cycle parameters.

### Study design

Double-blind, two parts, three to four cohorts (in each part), randomised, placebo-controlled study was carried out to investigate ascending /descending single doses **(Part A)** followed by ascending /descending multiple doses of E2MATE **(Part B)** comparing the safety, tolerability, pharmacokinetic and pharmacodynamic parameters of different E2MATE doses to placebo in healthy female subjects of reproductive age (mean age ranged from 30.8 to 35.2 years). Treatment was started within 72 hours after the beginning of menstrual bleeding.

**Part A:** Four cohorts of 8 subjects each investigating four different single doses of E2MATE administered orally using a sequential ascending design or a placebo. The active doses tested were 0.25 mg (1^{st} cohort), 1 mg, 4 mg and 8 mg.

**Part B:** Three cohorts of 8 subjects each investigating three different multiple doses of E2MATE administered orally on a weekly basis for four weeks, and using a sequential ascending design or a placebo. The active doses tested were 0.25 mg, 1 mg and 4 mg.

Blood samples were collected from each subject at several intervals after treatment start in order to determine pharmacodynamic parameters (sulfatase activity and hormonal profile). Ovarian and endometrium transvaginal ultrasounds throughout the study (Part A and B) and an endometrial biopsy (Part B only) were performed.

### Pharmacodynamic Variables

Pharmacodynamic properties were investigated by assessment of the STS activity in PBMCs and by measurement of the serum hormonal profile comprising the ratio of E1-S to E1 and DHEA-S to DHEA. STS activity was determined by spiking specimens with labelled E1-S and by measuring its conversion to E1 per gram protein under standardized incubation conditions using a validated radio scintillation assay. Protein concentration was evaluated with a validated colorimetric assay (Bradford method). Hormone profiles were analysed using commercially available ELISA or RIA kits.

### Results

### Single dose administration of 4 mg of E2MATE

Results showed that administration of a single dose of 4 mg E2MATE advantageously resulted in a nearly complete and long lasting STS inhibition in PBMCs. A nearly complete inhibition (96%) was reached already on Day 3 after treatment with 4 mg, which only slightly reduced during the study to 82% inhibition on Day 0 of cycle 2 (i.e. approximately 56 days after dosing). The average STS inhibition (over 56 days) was about 84% after treatment with single doses of 4 mg E2MATE compared to 23% to 27% after 0.25 mg and

1 mg E2MATE and placebo (Table 1 showing Maximal (Eₘₐₓ) and Average (AVG) Inhibition of Steroid Sulfatase in PBMCs after a single dose administration).

**Table 1**

| | | **0.25 mg (N=6)** | **1 mg (N=6)** | **4 mg (N=6)** | **Placebo (N=8)** |
|---|---|---|---|---|---|
| Eₘₐₓ (%) | Mean (SD) | 59.5 (30.2) | 58.7(10.9) | 99.5 (0.6) | 54.0 (31.5) |
| AVG (%) | Mean (SD) | 23.2 (14.6) | 25.2 (10.6) | 84.2 (6.3) | 26.6 (20.2) |

| | | | | | |
|---|---|---|---|---|---|
| Eₘₐₓ= maximal inhibitory effect = maximum of difference baseline - post-dose result. AVG = average effect = weighted average of differences baseline - post-dose result (calculated by trapezoidal rule, from Day 1 until end of study (EOS), divided by the length of the observation interval). | | | | | |

Single doses of 0.25 mg, 1 mg, 4 mg and 8 mg of E2MATE were well tolerated. No clinically relevant dose-related differences in the incidence of treatment emergent adverse events were observed. There were no clinically relevant changes in laboratory parameters, vital signs or ECG parameters.

After treatment with a single dose of 4 mg and 8 mg similar maximal increases in the E1-S/E1 ratio were observed (6.6 to 7.3). The ratios E1-S/E1 and DHEA-S/DHEA are consistent with the inhibition observed in leucocytes after single administration of the STS inhibitor.

No clinically relevant effects on the mean concentration-time profiles were observed for E2 (Table 2 showing maximal and weighted average estradiol concentrations), P4, LH, FSH, T and D4A after single dose administration of E2MATE compared to placebo.

**Table 2**

| | | **0.25 mg (N=6)** | **1 mg (N=6)** | **4 mg (N=6)** | **8 mg (N=6)** | **Placebo (N=8)** |
|---|---|---|---|---|---|---|
| Max (ng/L) | Mean | 335.1 | 318.1 | 275.3 | 221.1 | 232.2 |
| | SD | 178.6 | 188.9 | 109.1 | 100.2 | 92.9 |
| Avg (ng/L) | Mean | 141.2 | 131.0 | 113.8 | 109.3 | 102.5 |
| | SD | 40.7 | 67.5 | 31.4 | 33.8 | 32.2 |

Cycle length was not affected and all cycles were ovulatory except for cycle 2 in one subject each in the placebo and the 0.25 mg groups. No clinically relevant effects on the number of measurable follicles or on the number of subjects with follicles > 10 mm were observed. Moreover no clinically relevant differences between the treatment groups were seen for endometrial thickness assessed by TVUS on the different study days.

All pharmacodynamic parameters for E2MATE or EMATE increased dose-dependently after single dose administration, but the increase was more pronounced than expected for dose-proportionality.

### Multiple dose administration of 4 mg of E2MATE

These results showed that after multiple dose administration of 4 mg per week during four weeks nearly complete inhibition (82% to 100%) could be observed and maintained for the whole study period (Table 3 showing Maximal (Eₘₐₓ) and Average (AVG) Inhibition of Steroid Sulfatase in PBMCs after multiple dose administration).

**Table 3**

| | | **0.25 mg (N=5)** | **1 mg (N=6)** | **4 mg (N=6)** | **Placebo (N=6)** |
|---|---|---|---|---|---|
| Eₘₐₓ (%) | Mean (SD) | 42.0 (27.4) | 79.5 (25.6) | 100.0 (0.0) | 32.2 (22.9) |
| AVG (%) | Mean (SD) | 22.7 (16.5) | 43.5 (23.2) | 88.4 (9.5) | 11.7 (8.7) |

Multiple doses of 0.25, 1 mg and 4 mg E2MATE were well tolerated. No clinically relevant dose-related differences in the incidence of treatment emergent adverse events were observed. There were no clinically relevant changes in laboratory parameters, vital signs or ECG parameters.

A dose-dependent decreased turn-over of E1-S to E1 was observed and the increase in the E1-S/E1 ratio was highest after 4 mg on Day 14 and remained on this level until Day 0 of cycle 2. For the DHEA-S/DHEA ratios multiple doses of 1 mg and 4 mg E2MATE showed a similar maximal effect, but the increase in the ratio was faster in the 4 mg group and the effect lasted for longer. This is consistent with the inhibition observed in leucocytes after multiple administration of the STS inhibitor.

No clinically relevant dose-related differences in the mean concentration-time profiles were observed for E2 (Table 4 showing maximal and weighted average estradiol concentrations), P4, LH, FSH, T and D4A compared to placebo.

**Table 4**

| | | | 0.25 mg (N=6) | 1 mg (N=6) | 4mg (N=6) | Placebo (N=6) |
|---|---|---|---|---|---|---|
| Cycle 1 | Max (ng/L) | Mean | 305.3 | 278.6 | 317.8 | 284.8 |
| | | SD | 82.3 | 159.5 | 76.1 | 119.8 |
| | Avg (ng/L) | Mean | 135.7 | 126.0 | 134.8 | 117.5 |
| | | SD | 29.2 | 65.6 | 32.9 | 17.6 |
| Cycle 2 | Max (ng/L) | Mean | 260.5 | 193.4 | 207.7 | 198.2 |
| | | SD | 189.8 | 83.7 | 132.6 | 122.6 |
| | Avg (ng/L) | Mean | 116.0 | 109.0 | 100.9 | 98.5 |
| | | SD | 59.4 | 41.9 | 46.7 | 50.8 |
| Cycle 3 | Max (ng/L) | Mean | 185.4 | 221.6 | 180.9 | 191.8 |
| | | SD | 67.8 | 113.0 | 64.9 | 108.6 |
| | Avg (ng/L) | Mean | 107.1 | 110.0 | 94.9 | 103.2 |
| | | SD | 31.4 | 45.7 | 24.5 | 54.6 |

Cycle length was not affected and all cycles were ovulatory except for cycle 2 for in one subject each in the 4 mg group and in the placebo group and for cycle 3 in one subject each in the 1 mg and 4 mg groups.

Multiple doses of E2MATE had no clinically relevant effect on the number of visible follicles or on the number of subjects with follicles > 10 mm. Moreover no clinically relevant differences between the treatment groups were seen for endometrial thickness assessed by TVUS on the different study days.

Overall, all pharmacodynamic parameters for E2MATE or EMATE for after the first multiple dose were similar to those obtained after the single dose.

Finally, the correlation of Cₘₐₓ (maximum plasma drug concentration) and AUC (area under the plasma concentration-time curve) of E2MATE and EMATE showed a good correlation of the maximum and the average STS inhibition in PBMCs which was higher than after the single dose.

Therefore, altogether those results support the benefit of a regimen where the total dose of about 16 mg is reached after a treatment period of about four weeks and show that this regimen allows reaching a nearly complete STS inhibition which can be maintained for the whole study period without inducing any perturbation on the ovarian cycle parameters and circulating estradiol levels.

### Example 2: Dosage regimen with one loading period alone or in combination with a progestin

A dosage regimen of E2MATE according to the invention was carried out in healthy pre-menopausal women in order to support the benefit of a regimen according to the invention, notably in term of STS activity inhibition, circulating estradiol levels and ovarian cycle parameters.

### Study design

A randomised, double-blind, placebo-controlled Phase I study was carried out to evaluate the safety, tolerability, pharmacokinetics and pharmacodynamics of E2MATE administered alone (4 mg once per week) and in combination with norethisterone acetate (10 mg daily) for 4 weeks to healthy pre-menopausal women (mean age ranged from 18 to 40 years who have given vaginal birth at least once).

After an initial screening period for baseline measurements and endometrial biopsy to confirm eligibility, 24 subjects were randomised to one of three treatment groups (8 patients each) in a 1:1:1 ratio: **Treatment A** (E2MATE + NETA placebo), **Treatment B** (E2MATE placebo + NETA), **Treatment C** (E2MATE + NETA). Subjects received either E2MATE (4 mg) or matching E2MATE placebo on a weekly basis and NETA (10 mg) or NETA placebo daily for a duration of 4 weeks. E2MATE/placebo was administered under fasting conditions; for NETA/placebo no particular dietary conditions were necessary.

### Pharmacodynamic Variables (STS inhibition in endometrium and PBMCs and hormone profiles)

STS activity was determined by spiking specimens with labelled E1-S and by measuring its conversion to E1 per gram protein under standardized incubation conditions using LC-MS/MS. Protein concentration was evaluated with a validated colorimetric assay (Bradford method). Hormone profiles were determined as described in Example 1.

### Results

Multiple doses of 4 mg E2MATE alone and in combination with multiple doses of 10 mg NETA for 4 weeks were well tolerated in healthy pre-menopausal female subjects. There were no clinically relevant changes or significant findings in laboratory parameters, physical examinations, body weight, BMI, blood pressure, pulse rate, and ECG parameters. There was no indication of consistent differences in any of the safety and tolerability parameters evaluated between the three treatment groups.

### Pharmacodynamics

### STS activity in PBMCs

At baseline, mean STS activity was comparable between the three treatment groups. The mean percentage inhibition of STS activity on Day 4 was 90% and 89% in the E2MATE and E2MATE + NETA groups compared to 12% in the NETA group. During the following days, the percentage inhibition decreased in the E2MATE and E2MATE + NETA groups; on Day 113, the percentage inhibition was 37% and 63%, respectively. The mean maximal inhibition was similar for E2MATE and E2MATE + NETA (94.7% and 94.3%). For the average inhibition, again similar results were obtained for E2MATE and E2MATE + NETA (82.6% and 83.6%) and no inhibition was observed for the NETA group (4%). Those results are consistent with the multiple dosage of E2MATE at 4 mg from Example 1.

### STS activity in endometrium

Administration of 4 mg E2MATE alone or in combination with NETA resulted in a nearly complete and long lasting STS inhibition in endometrium. A nearly complete inhibition (E2MATE: 91% and E2MATE + NETA: 96%) was reached between Day 25 and Day 29, which only slightly reduced during the clinical trial to 88% and 93% inhibition on the third biopsy day (i.e. approximately 50 days after dosing). The mean maximal inhibition was similar for E2MATE and E2MATE + NETA (91.0% and 96.8%). For the average inhibition, similar results were obtained for the E2MATE and E2MATE +NETA groups as shown in Table 5 showing Maximal (Eₘₐₓ) and Average (AVG) Inhibition of Steroid Sulfatase in endometrium after multiple dose administration.

**Table 5**

| | **E2MATE (N = 8)** | **NETA (N=8)** | **E2MATE+NETA (N=8)** |
|---|---|---|---|
| | Mean (SD) | Mean (SD) | Mean (SD) |
| Eₘₐₓ [%] | 91.0 (2.6) | 46.7 (42.8) | 96.8 (4.0) |
| AVG [%] | 66.6 (2.7) | 14.9 (27.0) | 72.7 (2.8) |

Those results support the correlation between the STS activity in blood cells and in the endometrium.

STS inhibition was significantly higher after combined treatment with E2MATE + NETA (P<0.01) as well as 1 month after the end of the treatment (P<0.05). Similarly maximal observed effect and average effect over time were significantly higher after combined treatment (P<0.01) as well as summarized in Table 6 below showing the differences in STS inhibition in endometrium samples in the E2MATE and the E2MATE + NETA groups):

**Table 6**

| | **E2MATE** | **E2MATE+NETA** | **p-value** |
|---|---|---|---|
| STS inhibition [%] | 90.66 | 96.12 | 0.0066 |
| After treatment | 87.50 | 93.25 | 0.0391 |
| Eₘₐₓ (maximal observed effect) | 91.00 | 96.80 | 0.0041 |
| AVG (average effect over time (AUC)) | 66.60 | 72.70 | 0.0006 |

The variation observed at the end of treatment in subjects receiving NETA alone is considered to be an artefact due to NETA-induced endometrial thickness reduction leading to reduced biopsy quality.

### Functional biomarkers of STS activity (E1-S to E1 and DHEA-S to DHEA ratios)

This effect is confirmed on functional biomarkers of STS activity for at least 84 days. As described before, STS converts sulphate precursors of estrogens. Thus, by inhibiting STS activity a reduction in serum levels of estrone and DHEA and an increase in estrone sulphate and DHEA sulphate are expected. This effect is described by baseline corrected fold-changes of the E1-S to E1 and DHEA-S to DHEA ratios over time (study days).

Both E1 and E1S increases were observed after treatment with E2MATE and E2MATE + NETA with maximal effects between Day 15 and Day 29. For DHEA and DHEAS, a clear effect was observed (decrease or increase) after treatment with E2MATE in both treatment groups and a similar maximal increase compared to baseline was observed for the DHEAS/DHEA ratios. The effect still existed on Day 113.

The effect was again more pronounced in the E2MATE + NETA treatment group. During the NETA treatment period (values of days 8 to 29), STS dependent hormone conversions showed baseline relative mean increases of the E1-S to E1 ratio of up to 282% and 638% in the E2MATE and E2MATE + NETA groups, respectively, while DHEA-S to DHEA ratios increased up to 265% and 158%, respectively (Tables 7 below showing the differences of E1-S to E1 ratio in the E2MATE and the E2MATE+NETA groups and Table 8 showing the differences of DHEA-S to DHEA ratio in the E2MATE and the E2MATE+NETA groups).

**Table 7**

| **Baseline relative change of the E1-S / E1 [%]** | **E2MATE** | **E2MATE+NETA** | **p-value** |
|---|---|---|---|
| **Day** 1 | 0 | 0 | --- |
| **Day 8** | 208% | 461% | 0.10 |
| **Day 15** | 282% | 457% | 0.24 |
| **Day 22** | 218% | 638% | **0.03** |
| **Day 29** | 194% | 558% | **0.06** |
| **Day 57** | 218% | 342% | 0.17 |
| **Day 85** | 243% | 311% | 0.52 |
| **Day 113** | 400% | 260% | 0.46 |

**Table 8**

| **Baseline relative change of the DHEA-S / DHEA [%]** | **E2MATE** | **E2MATE+NETA** | **p-value** |
|---|---|---|---|
| Day 1 | 0 | 0 | --- |
| Day 8 | 133% | 156% | 0.58 |
| Day 15 | 116% | 157% | 0.40 |
| Day 22 | 113% | 235% | 0.03 |
| Day 29 | 148% | 265% | 0.08 |
| Day 57 | 195% | 202% | 0.93 |
| Day 85 | 232% | 249% | 0.88 |
| Day 113 | 175% | 202% | 0.71 |

Those results are supporting evidence of a synergic effect of E2MATE and NETA on functional biomarkers of STS activity reflecting the effects described in the endometrium.

### Estradiol and progesterone levels

Normal mean concentration-time profiles in E2 and P4 were observed after administration of E2MATE. After administration of NETA and E2MATE+NETA, E2 and P4 concentrations were suppressed during the first cycle up to Day 29 and a normal increase was observed on Day 43.

### Testosterone and androstenedione

No relevant differences in the mean concentration-time profiles were observed for T and D4A after multiple dose administration of E2MATE, NETA, or E2MATE + NETA.

### Follow-up effect of treatment on post-treatment menstruation cycles

The follow-up effect of the treatment with E2MATE and NETA on post-treatment menstrual cycles was assessed by evaluating the cycle duration, ovarian size, presence/absence of cysts, presence/absence of polycystic ovaries (only at screening), number of cysts > 30 mm, and by evaluating the endometrial thickness (measured by ovarian and transvaginal ultrasound). Menstruation cycle length was not influenced by the treatment. Administration of E2MATE, NETA, or E2MATE + NETA had no apparent effect on ovarian size. Sizes of right and left ovary differed slightly for each subject, but no clinically relevant changes were identified by the gynaecologists. Those data show that there was no distinct follow-up effect of the treatment on post-treatment menstrual cycles of the subjects.

### Conclusion

Altogether, those results confirm the results reported in Example 1 regarding the benefit of a regimen where the total dose of E2MATE of about 16 mg is reached after a loading treatment period of about four weeks and show that this regimen allows reaching a nearly complete STS inhibition in the endometrium which can be maintained for the whole study period without inducing any perturbation on the ovarian cycle parameters and circulating estradiol levels (in the group treated with E2MATE alone).

Further, those results surprisingly show that administration of E2MATE in a dosing regimen according to the invention in combination with a progestin (NETA) to healthy women of reproductive age elicit favourable pharmacodynamic responses demonstrating synergistic effects of E2MATE and NETA on STS activity inhibition. The synergic effect of NETA and E2MATE on STS inhibition in the target tissue (endometrium) described in this healthy female volunteers validates the concept that the combination of an administration regimen according to the invention of E2MATE with an administration combination with a progestin (e.g. NETA) is beneficial for the treatment of subjects suffering from endometriosis.

### Example 3: Dosage regimen simulation with one loading period followed by a maintenance period

Based on the results of Example 1 regarding the multiple dosing of E2MATE at a dose of 4 mg/week during 4 weeks (loading period), the accumulation ratio of E2MATE could be estimated and a pharmacokinetic model could be built to determine the maintenance dose required to maintain the blood levels of E2MATE at the level reached after the loading period.

As shown on Figure 2, the accumulation ratio (R_{acc}) based on the accumulation between two doses during one week time could be calculated. For example, the difference between the Cₘᵢₙ 504h (minimum steady-state plasma E2MATE + EMATE concentration during the dosage interval of 504 h) and Cₘᵢₙ, 168h (minimum steady-state plasma E2MATE + EMATE concentration during the dosage interval of 168 h) leads to an accumulation ratio about 2.7 which would be equivalent to 0.36 mg/day of E2MATE necessary to maintain the blood levels of E2MATE at the level reached after the loading period. This could be achieved for example by the administration of 1 mg every 3 days or 2 mg per week (Figure 2B).

As shown in Figure 3A, based on the STS inhibition in PBMCs from Example 1, in order to maintain the STS inhibition to 100% which is reached at the end of the loading period of E2MATE at a dose of 4 mg/week during 4 weeks, a concentration range of E2MATE + EMATE between 2'500-2'600 ng/mL should be maintained. This could be achieved for example by the administration of 1 mg every 3 days. Similarly, in order to maintain the STS inhibition to 95% after the end of the loading period of E2MATE at a dose of 4 mg/week during 4 weeks, a concentration range of E2MATE + EMATE between 2'200-2'500 ng/mL should be maintained which could be achieved for example by the administration of 2 mg per week.

The pharmacokinetic model generated based on the data of the study of Example 1 was used to simulate the plasma E2MATE + EMATE concentration for a loading period of administration of E2MATE at 4 mg/week for 4 weeks followed by a maintenance period of administration of E2MATE at 2 mg/week for 12 weeks (Figure 3B).

In order to validate this model, the pharmacokinetic data from the Example 2 were used and compared to those predicted by said model (Figure 3C). The results show a good prediction of the model in term of t_{1/2}, R_{acc} and Cₘₐₓ.

Therefore, the pharmacokinetic model supports that the pharmacokinetic and accumulation profile of E2MATE allows to maintain a nearly complete STS inhibition obtained at the end of a loading period where the total dose of about 16 mg is reached after a loading period of about four weeks, by the administration of reduced dose of E2MATE of about 1 to about 2 mg per week.

### Example 4: Dosage regimen with one loading period followed by a maintenance period

A dosage regimen of E2MATE according to the invention is carried out in women of reproductive age with a history of at least 3 months of non-menstrual pelvic pain and dysmenorrhea symptoms suggestive of endometriosis in order to support the benefit of a regimen according to the invention for the treatment of pain symptoms suggestive of endometriosis, notably in term of relief of pain symptoms where the steroid sulfatase inhibitor E2MATE is administered during two consecutive phases (i.e. loading phase and maintenance phase) with concomitant, continuous NETA administration.

Selected patients have not been treated by any hormonal treatment within the last 6 months, not having taken at any time any hormonal treatments (including OC pills continuously (28 day regimen), implants, progestins, GnRH agonists and antagonists or danazol) for the treatment of the suspected endometriosis or having undergone a surgical treatment for endometriosis within the last 12 months prior to screening.

The study is multicentre, randomised, two-arm, parallel group, double-blind, placebo controlled where eligible subjects were randomised on study day 1 in a 1:1 ratio into treatment group A or B between day 1 and day 4 of the menstrual bleeding.

Patients receive 4 mg of E2MATE (Group A) or matching placebo (Group B) once a week (4 tablets of 1mg) for 4 weeks, followed by 2 mg of E2MATE (2 tablets of 1mg) or matching placebo for 12 weeks, with concomitant oral administration of 1 tablet of 5 mg NETA once daily for 16 weeks, followed by NETA alone (same daily dose as previously) for 28 weeks. The baseline is established in terms of endometriosis profile regarding pelvic pain, dysmenorrhea, dyspaneuria and bleeding pattern and those parameters are followed during the treatment. During the loading phase the subjects are monitored on day 7, day 21 and on week 4 and during the maintenance phase monthly on week 8, week 12 and week 16. Pharmacodynamics (PD) of E2MATE, such as the inhibition of the STS activity in Peripheral Blood Mononuclear Cells (PBMCs) and the hormonal profile during 16 weeks of E2MATE treatment period and the long-term PD effect of E2MATE on the STS enzyme activity in PBMCs during 28 weeks follow-up period is investigated.

## Claims

1. E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks and wherein the total dose of E2MATE or EMATE reached at the end of the loading period is from about 8 to about 30 mg.

2. E2MATE or EMATE, any pharmaceutically acceptable salts or complexes thereof or a pharmaceutical formulation thereof for use in the treatment or prophylaxis of endometriosis according to claim 1, wherein E2MATE or any pharmaceutically acceptable salts or complexes thereof or a pharmaceutical formulation thereof is to be administered.

3. E2MATE or EMATE, any pharmaceutically acceptable salts or complexes thereof or a pharmaceutical formulation thereof for use in the treatment or prophylaxis of endometriosis according to claim 1 or 2, wherein E2MATE or EMATE, any pharmaceutically acceptable salts or complexes thereof or a pharmaceutical formulation thereof is to be administered orally.

4. E2MATE or EMATE, any pharmaceutically acceptable salts or complexes thereof or a pharmaceutical formulation thereof for use in the treatment or prophylaxis of endometriosis according to any one of claims 1 to 3, wherein E2MATE or EMATE, any pharmaceutically acceptable salts or complexes thereof or a pharmaceutical formulation thereof is to be administered every two or three days during the loading period.

5. E2MATE or EMATE, any pharmaceutically acceptable salts or complexes thereof or a pharmaceutical formulation thereof for use in the treatment or prophylaxis of endometriosis according to any one of claims 1 to 3, wherein E2MATE or EMATE, any pharmaceutically acceptable salts or complexes thereof or a pharmaceutical formulation thereof is to be administered once a week during the loading period.

6. E2MATE or EMATE, any pharmaceutically acceptable salts or complexes thereof or a pharmaceutical formulation thereof for use in the treatment or prophylaxis of endometriosis according to any one of claims 1 to 3, wherein said E2MATE or EMATE, any pharmaceutically acceptable salts thereof or a pharmaceutical formulation thereof is to be administered for at least one loading period lasting for about four weeks at a dose of about 4 mg/week during four weeks.

7. E2MATE or EMATE, any pharmaceutically acceptable salts or complexes thereof or a pharmaceutical formulation thereof for use in the treatment or prophylaxis of endometriosis according to any one of claims 1 to 6, wherein said E2MATE or EMATE, any pharmaceutically acceptable salts thereof or a pharmaceutical formulation thereof is to be administered is to be administered after the end of the loading period for at least one maintenance period at a dose of E2MATE or EMATE of 1 to about 3 mg/week.

8. E2MATE or EMATE, any pharmaceutically acceptable salts or complexes thereof or a pharmaceutical formulation thereof for use in the treatment or prophylaxis of endometriosis according to claim 7, wherein said E2MATE or EMATE, any pharmaceutically acceptable salts or complexes thereof or a pharmaceutical formulation thereof is to be administered once a week during the maintenance period.

9. E2MATE or EMATE, any pharmaceutically acceptable salts or complexes thereof or a pharmaceutical formulation thereof for use in the treatment or prophylaxis of endometriosis according to claim 7 or 8, wherein the maintenance period lasts for about 4 to 12 weeks.

10. E2MATE or EMATE, any pharmaceutically acceptable salts or complexes thereof or a pharmaceutical formulation thereof for use in the treatment or prophylaxis of endometriosis according to any one of claims 7 to 9, wherein E2MATE or EMATE, any pharmaceutically acceptable salts or complexes thereof or a pharmaceutical formulation thereof is to be administered once during the maintenance period at dose of about 2 mg/week.

11. E2MATE or EMATE, any pharmaceutically acceptable salts or complexes thereof or a pharmaceutical formulation thereof for use in the treatment or prophylaxis of endometriosis according to any one of claims 7 to 9, wherein E2MATE or EMATE, any pharmaceutically acceptable salts or complexes thereof or a pharmaceutical formulation thereof for use in the treatment or prophylaxis of endometriosis, wherein said E2MATE or EMATE, any pharmaceutically acceptable salts thereof or a pharmaceutical formulation thereof is to be administered for at least one loading period lasting for about four weeks at a dose of about 4 mg/week and is to be administered at the end of the loading period for at least one maintenance period lasting for at least about 4 weeks at a dose of about 2 mg/week.

12. E2MATE or EMATE, any pharmaceutically acceptable salts or complexes thereof or a pharmaceutical formulation thereof for use in the treatment or prophylaxis of endometriosis according to any one of claims 1 to 11, wherein a progestin, any pharmaceutically acceptable salts or complexes thereof or a pharmaceutical formulation thereof is to be administered in combination with E2MATE or EMATE, any pharmaceutically acceptable salts or complexes thereof or a pharmaceutical formulation thereof

13. E2MATE or EMATE, any pharmaceutically acceptable salts or complexes thereof or a pharmaceutical formulation thereof for use in the treatment or prophylaxis of endometriosis according to claim 12, wherein a progestin is to be administered during the loading and/or maintenance period at a daily dose of about 0.05 to about 100 mg/day in combination with E2MATE or EMATE, any pharmaceutically acceptable salts or complexes thereof or a pharmaceutical formulation thereof.

14. E2MATE or EMATE, any pharmaceutically acceptable salts or complexes thereof or a pharmaceutical formulation thereof for use in the treatment or prophylaxis of endometriosis according to claim 12 or 13, wherein norethisterone (NET) or norethindrone (norethisterone) acetate (NETA) any pharmaceutically acceptable salts or complexes thereof or a pharmaceutical formulation thereof is to be administered in combination with E2MATE or EMATE, any pharmaceutically acceptable salts or complexes thereof or a pharmaceutical formulation thereof.

15. A pharmaceutical pack or kit comprising one or more containers filled with E2MATE or EMATE, any pharmaceutically acceptable salts or complexes thereof or a pharmaceutical formulation thereof, wherein each container contains a necessary single dose for a treatment lasting about four weeks at a total dose of E2MATE or EMATE of about 8 to about 30 mg.
